# EUROPEAN PATENT APPLICATION

(11) **EP 0 739 982 A1**
(43) Date of publication of application: **30.10.1996**
(21) Application number: 95201115.3
(22) Date of filing: 28.04.1995
(51) Int. Cl.: C12N 15/56, C12N 15/63, C12N 9/42, C12N 1/21, C11D 3/386

(54) **Bacillus cellulase and its applications**

(71) Applicant: GENENCOR INTERNATIONAL, INC., Rochester, New York 14618 (US)
(72) Inventor: van Beckhoven, Rudolf Franciscus Wilhelmus C., NL-4334 EK Breda (NL); Lenting, Hermanus Bernardus Maria, NL-2641 VT Pijnacker (NL); Maurer, Karl-Heinz, D-40699 Erkrath (DE); van Solingen, Pieter, NL-2671 VZ Naaldwijk (NL); Weiss, Albrecht, D-40764 Langenfeld (DE)
(74) Representative: Matulewicz, Emil Rudolf Antonius, Dr.

(57) **Abstract**

The present invention discloses an enzyme having cellulase activity. The single cellulase is characterized in that it is able to provide both antiredeposition and depilling effects when applied in laundry washing. The enzyme is obtainable from a deposited strain of the genus Bacillus. The enzyme is suited for use in detergent- and textile-treatment applications.

SEQUENCE LISTING

(1) GENERAL INFORMATION:

(i) APPLICANT:
(A) NAME: Gist-brocades
(B) STREET: Wateringseweg 1
(C) CITY: Delft
(E) COUNTRY: The Netherlands
(F) POSTAL CODE (ZIP): 2611 XT

(ii) TITLE OF INVENTION: Novel Cellulase and Its
Applications

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 1404 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Bacillus sp.
(C) INDIVIDUAL ISOLATE: CBS 670.93

(ix) FEATURE:
(A) NAME/KEY: sig_peptide
(B) LOCATION: 1..78

(ix) FEATURE:
(A) NAME/KEY: mat_peptide
(B) LOCATION: 79..1404
(D) OTHER INFORMATION: /function= "endoglucanase"
/EC_number= 3.2.1.4
/product= "BCE103 cellulase"

(ix) FEATURE:
(A) NAME/KEY: CDS
(B) LOCATION: 1..1404

(xi) SEQUENCE DESCRIPTION: SEQ ID NO: 1:

ATG AAA AAG ATA ACT ACT ATT TTT GCC GTA TTG CTC ATG ACA TTG GCG 48
Met Lys Lys Ile Thr Thr Ile Phe Ala Val Leu Leu Met Thr Leu Ala
-26 -25 -20 -15

TTG TTC AGT ATA GGA AAC ACG ACA GCG GCT GAT GAT TAT TCA GTT GTA 96
Leu Phe Ser Ile Gly Asn Thr Thr Ala Ala Asp Asp Tyr Ser Val Val
-10 -5 1 5

GAG GAA CAT GGG CAA CTA AGT ATT AGT AAC GGT GAA TTA GTC AAT GAA 144
Glu Glu His Gly Gln Leu Ser Ile Ser Asn Gly Glu Leu Val Asn Glu
10 15 20

CGA GGC GAA CAA GTT CAG TTA AAA GGG ATG AGT TCC CAT GGT TTG CAA 192
Arg Gly Glu Gln Val Gln Leu Lys Gly Met Ser Ser His Gly Leu Gln
25 30 35

TGG TAC GGT CAA TTT GTA AAC TAT GAA AGC ATG AAA TGG CTA AGA GAT 240
Trp Tyr Gly Gln Phe Val Asn Tyr Glu Ser Met Lys Trp Leu Arg Asp
40 45 50

GAT TGG GGA ATA ACT GTA TTC CGA GCA GCA ATG TAT ACC TCT TCA GGA 288
Asp Trp Gly Ile Thr Val Phe Arg Ala Ala Met Tyr Thr Ser Ser Gly
55 60 65 70

GGA TAT ATT GAC GAT CCA TCA GTA AAG GAA AAA GTA AAA GAG ACT GTT 336
Gly Tyr Ile Asp Asp Pro Ser Val Lys Glu Lys Val Lys Glu Thr Val
75 80 85

GAG GCT GCG ATA GAC CTT GGC ATA TAT GTG ATC ATT GAT TGG CAT ATC 384
Glu Ala Ala Ile Asp Leu Gly Ile Tyr Val Ile Ile Asp Trp His Ile
90 95 100

CTT TCA GAC AAT GAC CCG AAT ATA TAT AAA GAA GAA GCG AAG GAT TTC 432
Leu Ser Asp Asn Asp Pro Asn Ile Tyr Lys Glu Glu Ala Lys Asp Phe
105 110 115

TTT GAT GAA ATG TCA GAG TTG TAT GGA GAC TAT CCG AAT GTG ATA TAC 480
Phe Asp Glu Met Ser Glu Leu Tyr Gly Asp Tyr Pro Asn Val Ile Tyr
120 125 130

GAA ATT GCA AAT GAA CCG AAT GGT AGT GAT GTT ACG TGG GAC AAT CAA 528
Glu Ile Ala Asn Glu Pro Asn Gly Ser Asp Val Thr Trp Asp Asn Gln
135 140 145 150

ATA AAA CCG TAT GCA GAA GAA GTG ATT CCG GTT ATT CGT GAC AAT GAC 576
Ile Lys Pro Tyr Ala Glu Glu Val Ile Pro Val Ile Arg Asp Asn Asp
155 160 165

CCT AAT AAC ATT GTT ATT GTA GGT ACA GGT ACA TGG AGT CAG GAT GTC 624
Pro Asn Asn Ile Val Ile Val Gly Thr Gly Thr Trp Ser Gln Asp Val
170 175 180

CAT CAT GCA GCC GAT AAT CAG CTT GCA GAT CCT AAC GTC ATG TAT GCA 672
His His Ala Ala Asp Asn Gln Leu Ala Asp Pro Asn Val Met Tyr Ala
185 190 195

TTT CAT TTT TAT GCA GGA ACA CAT GGA CAA AAT TTA CGA GAC CAA GTA 720
Phe His Phe Tyr Ala Gly Thr His Gly Gln Asn Leu Arg Asp Gln Val
200 205 210

GAT TAT GCA TTA GAT CAA GGA GCA GCG ATA TTT GTT AGT GAA TGG GGG 768
Asp Tyr Ala Leu Asp Gln Gly Ala Ala Ile Phe Val Ser Glu Trp Gly
215 220 225 230

ACA AGT GCA GCT ACA GGT GAT GGT GGT GTG TTT TTA GAT GAA GCA CAA 816
Thr Ser Ala Ala Thr Gly Asp Gly Gly Val Phe Leu Asp Glu Ala Gln
235 240 245

GTG TGG ATT GAC TTT ATG GAT GAA AGA AAT TTA AGC TGG GCC AAC TGG 864
Val Trp Ile Asp Phe Met Asp Glu Arg Asn Leu Ser Trp Ala Asn Trp
250 255 260

TCT CTA ACG CAT AAG GAT GAG TCA TCT GCA GCG TTA ATG CCA GGT GCA 912
Ser Leu Thr His Lys Asp Glu Ser Ser Ala Ala Leu Met Pro Gly Ala
265 270 275

AAT CCA ACT GGT GGT TGG ACA GAG GCT GAA CTA TCT CCA TCT GGT ACA 960
Asn Pro Thr Gly Gly Trp Thr Glu Ala Glu Leu Ser Pro Ser Gly Thr
280 285 290

TTT GTG AGG GAA AAA ATA AGA GAA TCA GCA TCT ATT CCG CCA AGC GAT 1008
Phe Val Arg Glu Lys Ile Arg Glu Ser Ala Ser Ile Pro Pro Ser Asp
295 300 305 310

CCA ACA CCG CCA TCT GAT CCA GGA GAA CCG GAT CCA GGA GAA CCG GAT 1056
Pro Thr Pro Pro Ser Asp Pro Gly Glu Pro Asp Pro Gly Glu Pro Asp
315 320 325

CCA ACG CCC CCA AGT GAT CCA GGA GAG TAT CCA GCA TGG GAT TCA AAT 1104
Pro Thr Pro Pro Ser Asp Pro Gly Glu Tyr Pro Ala Trp Asp Ser Asn
330 335 340

CAA ATT TAC ACA AAT GAA ATT GTG TAT CAT AAC GGT CAG TTA TGG CAA 1152
Gln Ile Tyr Thr Asn Glu Ile Val Tyr His Asn Gly Gln Leu Trp Gln
345 350 355

GCG AAA TGG TGG ACA CAA AAT CAA GAG CCA GGT GAC CCA TAC GGT CCG 1200
Ala Lys Trp Trp Thr Gln Asn Gln Glu Pro Gly Asp Pro Tyr Gly Pro
360 365 370

TGG GAA CCA CTC AAA TCT GAC CCA GAT TCA GGA GAA CCG GAT CCA ACG 1248
Trp Glu Pro Leu Lys Ser Asp Pro Asp Ser Gly Glu Pro Asp Pro Thr
375 380 385 390

CCC CCA AGT GAT CCA GGA GAG TAT CCA GCA TGG GAT TCA AAT CAA ATT 1296
Pro Pro Ser Asp Pro Gly Glu Tyr Pro Ala Trp Asp Ser Asn Gln Ile
395 400 405

TAC ACA AAT GAA ATT GTG TAC CAT AAC GGC CAG CTA TGG CAA GCA AAA 1344
Tyr Thr Asn Glu Ile Val Tyr His Asn Gly Gln Leu Trp Gln Ala Lys
410 415 420

TGG TGG ACA CAA AAT CAA GAG CCA GGT GAC CCA TAT GGT CCG TGG GAA 1392
Trp Trp Thr Gln Asn Gln Glu Pro Gly Asp Pro Tyr Gly Pro Trp Glu
425 430 435

CCA CTC AAT TAA 1404
Pro Leu Asn
440

(2) INFORMATION FOR SEQ ID NO: 2:

(i) SEQUENCE CHARACTERISTICS:
(A) LENGTH: 467 amino acids
(B) TYPE: amino acid
(D) TOPOLOGY: linear

(ii) MOLECULE TYPE: protein

(xi) SEQUENCE DESCRIPTION: SEQ ID NO: 2:

Met Lys Lys Ile Thr Thr Ile Phe Ala Val Leu Leu Met Thr Leu Ala
-26 -25 -20 -15

Leu Phe Ser Ile Gly Asn Thr Thr Ala Ala Asp Asp Tyr Ser Val Val
-10 -5 1 5

Glu Glu His Gly Gln Leu Ser Ile Ser Asn Gly Glu Leu Val Asn Glu
10 15 20

Arg Gly Glu Gln Val Gln Leu Lys Gly Met Ser Ser His Gly Leu Gln
25 30 35

Trp Tyr Gly Gln Phe Val Asn Tyr Glu Ser Met Lys Trp Leu Arg Asp
    40                  45                  50

Asp Trp Gly Ile Thr Val Phe Arg Ala Ala Met Tyr Thr Ser Ser Gly
55                  60                  65                  70

Gly Tyr Ile Asp Asp Pro Ser Val Lys Glu Lys Val Lys Glu Thr Val
                75                  80                  85

Glu Ala Ala Ile Asp Leu Gly Ile Tyr Val Ile Ile Asp Trp His Ile
            90                  95                  100

Leu Ser Asp Asn Asp Pro Asn Ile Tyr Lys Glu Glu Ala Lys Asp Phe
        105                 110                 115

Phe Asp Glu Met Ser Glu Leu Tyr Gly Asp Tyr Pro Asn Val Ile Tyr
    120                 125                 130

Glu Ile Ala Asn Glu Pro Asn Gly Ser Asp Val Thr Trp Asp Asn Gln
135                 140                 145                 150

Ile Lys Pro Tyr Ala Glu Glu Val Ile Pro Val Ile Arg Asp Asn Asp
                155                 160                 165

Pro Asn Asn Ile Val Ile Val Gly Thr Gly Thr Trp Ser Gln Asp Val
            170                 175                 180

His His Ala Ala Asp Asn Gln Leu Ala Asp Pro Asn Val Met Tyr Ala
        185                 190                 195

Phe His Phe Tyr Ala Gly Thr His Gly Gln Asn Leu Arg Asp Gln Val
    200                 205                 210

Asp Tyr Ala Leu Asp Gln Gly Ala Ala Ile Phe Val Ser Glu Trp Gly
215                 220                 225                 230

Thr Ser Ala Ala Thr Gly Asp Gly Gly Val Phe Leu Asp Glu Ala Gln
                235                 240                 245

Val Trp Ile Asp Phe Met Asp Glu Arg Asn Leu Ser Trp Ala Asn Trp
            250                 255                 260

Ser Leu Thr His Lys Asp Glu Ser Ser Ala Ala Leu Met Pro Gly Ala
        265                 270                 275

Asn Pro Thr Gly Gly Trp Thr Glu Ala Glu Leu Ser Pro Ser Gly Thr
    280                 285                 290

Phe Val Arg Glu Lys Ile Arg Glu Ser Ala Ser Ile Pro Pro Ser Asp
295                 300                 305                 310

Pro Thr Pro Pro Ser Asp Pro Gly Glu Pro Asp Pro Gly Glu Pro Asp
                315                 320                 325

Pro Thr Pro Pro Ser Asp Pro Gly Glu Tyr Pro Ala Trp Asp Ser Asn
            330                 335                 340

Gln Ile Tyr Thr Asn Glu Ile Val Tyr His Asn Gly Gln Leu Trp Gln
        345                 350                 355

Ala Lys Trp Trp Thr Gln Asn Gln Glu Pro Gly Asp Pro Tyr Gly Pro
    360                 365                 370

Trp Glu Pro Leu Lys Ser Asp Pro Asp Ser Gly Glu Pro Asp Pro Thr
375                 380                 385                 390

Pro Pro Ser Asp Pro Gly Glu Tyr Pro Ala Trp Asp Ser Asn Gln Ile
                395                 400                 405
Tyr Thr Asn Glu Ile Val Tyr His Asn Gly Gln Leu Trp Gln Ala Lys
            410                 415                 420
Trp Trp Thr Gln Asn Gln Glu Pro Gly Asp Pro Tyr Gly Pro Trp Glu
    425                 430                 435
Pro Leu Asn
    440

## Description

### Technical field

The present invention relates to a novel cellulase. The invention further relates to detergent additives comprising the novel cellulase and to detergent compositions containing the novel cellulase. The invention also relates to the use of the novel cellulase in the treatment of cotton containing fabrics.

### Background of the invention

Cellulases, also called cellulolytic enzymes, are enzymes which are capable of the hydrolysis of the β-D-glucosidic linkages in celluloses. Cellulolytic enzymes have been divided traditionally into three classes: endoglucanases, exoglucanases or cellobiohydrolases and β-glucosidases (Knowles, J. et al. (1987), TIBTECH 5, 255-261). Cellulolytic enzymes can be produced by a large number of bacteria, yeasts and fungi. Microorganisms that produce cellulases are for example described in GB 2094826 (Kao Corporation).

Several applications have been developed for use of cellulolytic enzymes:
- degrading (wood)cellulose pulp into sugars for (bio)ethanol production;
- several textile treatments like 'stone washing' and 'biopolishing';
- application in detergent compositions.
The use of cellulases in detergent compositions started with cellulases capable of reducing the harshness (softening) of cotton containing fabrics (GB 1358599 (Unilever)).

It is further known that detergent compositions comprising cellulases are effective in removing dirt (cleaning). The efficiency of cellulolytic enzymes, cellulases, in terms of cleaning textile has been recognized for some time; GB-A-2075028, GB-A-2095275 and GB-A-2094826 (Kao Corporation) disclose detergent compositions with cellulase for improved cleaning performance.

It is also known in the art that cellulases can act as a colour clarifying agent in laundry detergents. After repeated washing of soiled fabrics, cotton containing fabrics appear to be greyish, most probably due to disrupted fibres caused by mechanical action and causes the greyish appearance of coloured cotton containing fabrics. The fibres are torn up resulting in disordered fibres which are broken. The use of cellulases as colour clarification agents for coloured fabrics has been described in EP-A-0220016 (Novo-Nordisk).

The main disadvantage of the cellulases known in the art showing colour clarification is that these enzymes agressively degrade the cellulose containing fabrics which results in undesirable loss of tensile strength of the fabrics.

On the other hand cellulases known to the art showing good cleaning properties show hardly any colour clarification effects.

From the above it will become clear that it is still desirable to provide for improved cellulases in detergent applications.

### Summary of the invention

The present invention relates to a novel (single) cellulase which cellulase exhibits the following properties:
(a) show a delta REM of at least 4 units in the Anti Redeposition test, and
(b) show a depilling result which depilling result is at least comparable to that of the cellulase obtainable from Bacillus sp. CBS 670.93 in the Depilling test.

Surprisingly it has been found that there are (single) cellulases which are capable of both cleaning, antiredeposition, colour clarification (by depilling action of the cellulase) and pilling prevention (antipilling) performance in laundry washing, obtainable from microorganisms.

Mixtures of cellulases as suggested in WO 95/02675 (Novo and Procter & Gamble) and known mixtures of cellulases like Celluzyme® (Novo) were known to provide the above mentioned performances in laundry washing, but single enzymes providing all these characteristics when applied in laundry washing are novel.

It is further found that the (single) cellulase of the invention, unlike previously known mixtures of cellulases which provide colour clarification, do not degrade cotton to an undesirable level causing tensile strength loss.

It is further found that this cellulase unlike previously known cellulases which provide colour clarification, do not accumulate on the fabric after repeated laundry washing.

The invention further provides a process for producing such a novel cellulase.

In another aspect, the invention provides detergent compositions, detergent additives, fabric softeners and depilling compositions comprising the novel cellulase.

Still another aspect of the invention is the use of the novel cellulases in methods for treating cotton containing textiles, like 'Stone wash' and 'Biopolish' processes.

### Legend to the figure

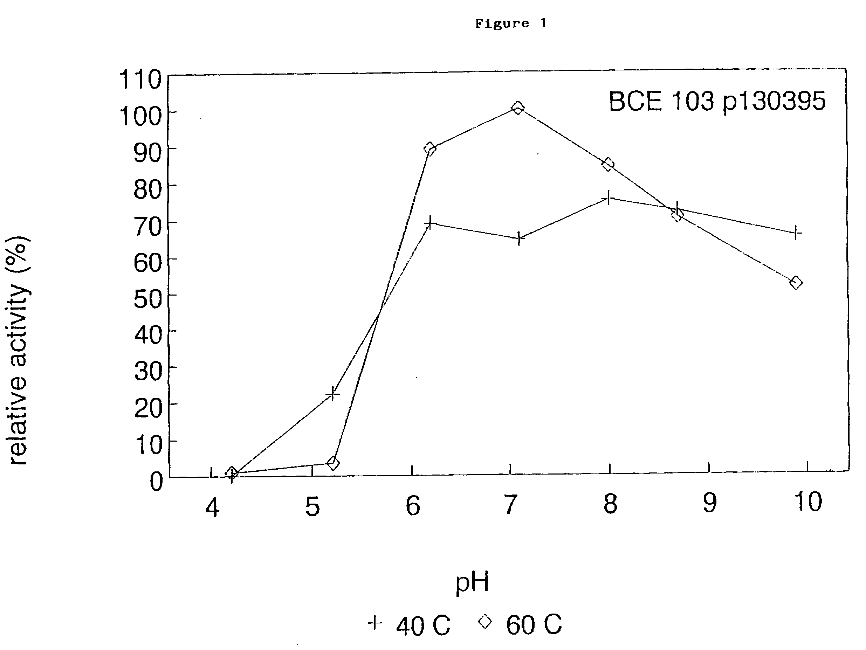

Figure 1 shows the relative activities of the cellulase obtainable from E. coli clone BCE 103. In Example 3 this figure is referred to as the pH/temperature profiles. All activities for both 40 and 60°C are related to the highest activity which is fixed on 100%.

### Detailed disclosure of the invention

As noted above, the present invention generally relates to a novel cellulase and its applications. However, prior to disclosing this invention in detail, first the following terms will be defined.

"Cellulase" is a generic name for enzymes acting on cellulose and its derivatives, and hydrolysing them into glucose, cellobiose or cellooligosaccharides.

The term "single" cellulase used herein is intended to mean a cellulase which is produced by one gene.

The term "cleaning" means the removal of dirt attached to laundry.

The term "pilling" in this respect is the formation of pills and fuzz on the surface of cotton containing fabrics due to broken or disordered fibres.

The term "depilling" is the removal of pills and fuzz from cotton containing fabrics. Depilling results in colour clarification when coloured cotton containing fabrics are depilled.

The term "colour clarification" in this respect is the reestablishment of the attractive fresh look of coloured fabrics containing or consisting of cellulose based fibres, which have developed a greyish appearance by a cellulase treatment of the coloured fabric.

The term "antiredeposition" in this respect is the action of cellulase to prevent or diminish the redeposition of dirt and colour components on the fabric.

The term "redeposition" in this respect is deposition of dirt or colour components that were removed from these textiles or fabrics during a laundry washing or textile treatment.

The term "derivative" is intended to indicate a protein which is derived from the native protein by addition of one or more amino acids to either or both the C- and N-terminal end of the native protein, substitution of one or more amino acids at one or a number of different sites in the native amino acid sequence, deletion of one or more amino acids at either or both ends of the native protein or at one or more sites in the amino acid sequence, or insertion of one or more amino acids at one or more sites in the native amino acid sequence.

The present invention relates to a novel cellulase which is obtainable from the following microorganism which is deposited according to the Budapest Treaty on the International Recognition of the Deposits of Microorganisms for the Purposes of Patent Procedures, at the Centraal Bureau voor Schimmelcultures, Baarn, The Netherlands on December 23, 1993 under deposition number CBS 670.93 (already described in copending application PCT/EP94/04312). This strain is classified as a new species of the genus Bacillus, which does not belong to any of the presently known rRNA-groups of Bacillus.

The microorganism may be obtained for example from water and soil samples collected in alkaline environments such as alkaline soils and soda lakes.

The microorganisms have subsequently been screened using a carboxymethyl cellulose (CMC)-agar diffusion assay. Strains which showed a clearing zone in this test were isolated as potential cellulase producing strains. Genomic gene libraries of the alkali tolerant cellulase producing strains were constructed. Recombinant clones were screened by agar diffusion on CMC-agar. Recombinant clones that showed clearing zones around the colony were isolated. Single cellulases were produced by fermentation of the recombinant clones in 4*YEP-medium for 48 hours at 30°C. The obtained single cellulases optionally purified as described in Example 2 were tested in the following tests:
a) Anti redeposition test;
b) Depilling test.
Surprisingly we have found that the cellulase obtainable from CBS 670.93 shows a good performance in both tests.

The present invention discloses a cellulase which cellulase exhibit the following properties:
(a) show a delta REM of at least 4 units in the Anti Redeposition Test and
(b) show a depilling result which depilling result is at least comparable to that of the cellulase obtainable from Bacillus sp. CBS 670.93 in the Depilling Test.

The Anti Redeposition Test is described in Example 4. Whiteness maintenance of white fabric is measured by a reflectance measurement. The higher the reflectance value, the more effective is the tested cellulase in antiredeposition performance.

The Depilling Test is described in Example 5. Depilling is the removal of fibres that are disordered and/or broken which make the coloured cotton containing fabric look greyish. The more disordered and/or broken fibres are removed the better the coloured cotton containing fabrics look. Depilling effectiveness can be judged by panels or can be quantified by an image analysis system. In the image analysis system an area of 4x4 cm is transferred from a surface microscope to a CCD camera connected to an image analyzing system. The pilling is indicated as a percentage of white area on the dark area of unpilled textile. The results can be given as a percentage of pilled area.

The cellulase of the present invention was further characterized by the Fibre Damage test. This test is described in Example 4. Surprisingly it has been found that the cellulase of the invention, while showing a good depilling effect does not show much fibre damage. The fibre damage can further be quantified by a tensile strength test, as described in International Standard ISO 2267.

The cellulase of the present invention was even further characterized by the Adsorption test. This test is described in Example 4. Low adsorption of the cellulase to the cotton is desired. Surprisingly it has been found that the cellulase of the invention, while showing a good depilling effect do not adsorb to the cotton as much as previously known depilling cellulases do.

Results from small scale experiments can be confirmed by full scale laundry washing experiments. The cellulase of the present invention can further be characterized by the pH and temperature activity profiles. These profiles can be made by using the CMC'ase assay as described in Example 3. By varying the pH or temperature at the enzyme incubation, pH and temperature profiles can be obtained. For determination of the pH profile a phosphate/citrate buffer system can be used.

The present invention also discloses a process for the production of the cellulase of the present invention, which can be developed using genetic engineering. As a first step the gene encoding the cellulase of the present invention can be cloned using λ-phage (expression) vectors and E. coli host cells. (Alternatively PCR cloning using consensus primers designed on conserved domains may be used.) Expression of the gene encoding the cellulase of the present invention in E. coli, is shown to give an active protein.

After a first cloning step in E. coli, a cellulase gene can be transferred to a more preferred industrial expression host such as Bacillus or Streptomyces species, a filamentous fungus such as Aspergillus, or a yeast. High level expression and secretion obtainable in these host organisms allows accumulation of the cellulase of the invention in the fermentation medium from which they can subsequently be recovered.

The present invention further relates to a detergent composition which comprises the above described cellulase.

Detergent compositions comprising the inventive cellulase may additionally comprise surfactants which may be of the anionic, non-ionic, cationic, amphoteric or zwitterionic type as well as mixtures of these surfactant classes. Examples of surfactants are described in GB 2094826-A (Kao Corporation).

Detergent compositions of the invention may contain other detergent ingredients known in the art as e.g. builders, bleaching agents, bleach activators, anti-corrosion agents, sequestering agents, anti soil-redeposition agents, perfumes, enzyme stabilizers, etc.

The detergent compositions of the invention may be formulated in any convenient form e.g. as a powder or liquid.

Fabric softening compositions comprising the inventive cellulase may further comprise cationic surfactants which are capable of fabric softening.

Depilling compositions comprising the inventive cellulase may further comprise surfactants and/or pH stabilizers. Depilling compositions can be used for a one time treatment of cotton containing fabrics.

The present invention further relates to the use of the above described cellulase in wet processing of cellulosic-based fabrics or garments.

For example cellulases are used to give an abraded look to ring dyed cellulosic goods, in particular to achieve variations in colour density in indigo-dyed denim products providing the popular stonewashed look of denim jeans (described in EP 307564). The cellulases replace or reduce the amount of pumice stones that used to be applied in jeans manufacturing.

Another example is the "Biopolish" process (described by Asferg et al. (1990), Int. Textile Bulletin, Dyeing/Printing/Finishing, 36, 5-8) in which cellulases are applied to improve the quality of cellulosic fabrics. Typical biopolish effects are for example a better feel & handling, decreased amount of fuzz, lower tendency to form pills (pilling prevention), improved softness or increased luster in comparison to the untreated fabric.

It has been found that the cellulase of the present invention is capable of both providing a Stone Washed effect and a Biopolish effect when used in these processes.

The invention will be explained in more detail in the following examples which are provided for illustration and are not to be construed as limiting on the invention.

### Example 1

### Screening for cellulase producing microorganisms

Two methods were applied for the isolation of cellulase-producing microorganisms:
i) the soil and water samples were suspended in 0.85% saline solution and directly used in the carboxymethyl cellulose (CMC)-agar diffusion assay for detection of cellulase producing colonies.
ii) The soil and water samples were enriched for cellulase containing strains by incubation in a cellulose containing liquid minimal medium or GAM-medium for 1 to 3 days at 40°C. Cultures that showed bacterial growth were analyzed for cellulase activity using the CMC-agar diffusion assay for detection of cellulase producing colonies.

### Isolation of alkalitolerant, cellulase producing strains

Strains that showed clearing zones in the agar diffusion assay were fermented in 25 millilitre GAM-medium in 100 millilitre shake flasks in an Incubator Shaker (New Brunswick Scientific, Edison, NJ, USA), at 250 r.p.m. at 40°C for 72 hours. CMCase activity was determined in the culture broth at pH 9 and 40°C.

### Isolation of cellulase genes

Genomic gene libraries of the alkalitolerant cellulase producing strains were constructed in plasmid pTZ18R (Mead, D.A., et al. (1986) Protein Engineering 1, 67). Recombinant clones were screened by agar diffusion on CMC-agar as described by Wood, P.J., et al. (1988) Methods in Enzymology 160, 59-74. Strains that showed clearing zones around the colony were isolated. The CMCase activity of the recombinant strains was determined after fermentation for 48 hours at 30°C in 4*YEP-medium. The plasmid DNA of the recombinant strains was isolated and the inserts were characterized by restriction enzyme analysis and nucleotide sequence analysis.

### Media

The minimal medium (pH 9.7) used in the CMC-agar diffusion assay and the enrichment procedure, consisted of KNO₃ 1%, Yeast extract (Difco) 0.1%, KH₂PO₄ 0.1%, MgSO₄.7H₂O 0.02%, Na₂CO₃ 1%, NaCl 4% and 0.25% CMC (Sigma C-4888). For solidificaton 1.5% agar was added.

The complex medium (GAM) used for enzyme production of the donor strains consisted of Peptone (Difco) 0.5%, Yeast extract (Difco) 0.5%, Glucose.H₂O 1%, KH₂PO₄ 0.1%, MgSO₄.7H₂O 0.02%, Na₂CO₃ 1%, NaCl 4%. The pH was adjusted to 9.5 with 4M HCl after which 1% CMC was added.

The complex medium (4*YEP) used for the enzyme production in E. coli recombinant strains consisted of Yeast extract (Difco) 4%, Peptone (Difco) 8%, lactose 0.2%, 100 µg/ml ampicilline).

### CMC-agar diffusion assay for colonies

Cell suspensions in 0.85% saline solution were plated on CMC-containing minimal medium. After incubation for 1 to 3 days at 40°C, the plates were replica plated and the parent plate was flooded with 0.1% Congo Red for 15 minutes. The plates were destained with 1M NaCl for 30 minutes. The strains that showed a clearing zone aroung the colony were isolated as potential cellulases producing microorganisms.

### CMC-agar diffusion assay for liquid fractions

Aliquots of 40 µl of enzyme solution or fermentation broth were pipetted in wells punched out from a layer of 5 mm of minimal medium in a petri dish. After incubation for 16 hours at 40°C cellulase activity was detected by Congo Red / NaCl treatment. The diameter of the clearing zone is a measure for the CMCase activity.

### Results of Example 1

The experiments of Example 1 resulted in the isolation of a cellulase producing microorganism which was deposited thereafter as CBS 670.93. The microorganism was classified as a new species of the genus Bacillus. Cloning experiments of Example 1 with the CBS 670.93 strain as a donor strain resulted in the isolation of an E. coli clone called BCE 103 which was able to produce a cellulase. The nucleotide sequence of the gene coding for said cellulase was analysed. From the cellulase produced by BCE 103 the N-terminal amino acid sequence was determined using standard methods for obtaining and sequencing peptides (Finlay & Geisow (Eds.), Protein Sequencing - a practical approach, 1989, IRL Press). The amino acid sequence of the cellulase was deduced from the nucleotide sequence, using the N-terminal amino acid sequence for the starting point of the mature protein.

The nucleotide sequence is shown in SEQ ID No. 1 and the amino acid sequence is shown in SEQ ID No. 2.

### Example 2

### Purification of cellulases

After the fermentation the cells were separated from the culture liquid by centrifugation (8000 rpm). The cellulase in the supernatant was precipitated with ammonium sulphate (65% saturation). The precipitate was dissolved in 25 mM phosphate buffer pH 7 + 5 mM EDTA until a conductivity of 7 mS/cm. This solution was applied to a Q-Sepharose FF (diameter 5 cm, length 10 cm) Anion Exchange column, after which the column was washed with 25 mM phosphate buffer pH 7 + 5 mM EDTA until an absorbency of 0.2 AU. A gradient of 0 to 0.5 M NaCl in 25 mM phosphate pH 7 was applied to the column in 80 minutes followed by a gradient from 0.5 to 1 M NaCl in 10 minutes. Depending on which cellulase was applied to the column, elution took place in the first or the second gradient. After elution the column was cleaned (upflow) with 1 M NaOH and equilibrated again with 25 mM phosphate pH 7 + 5 mM EDTA.

Depending on the elution the obtained cellulase had a purity of up to about 80%.

### Example 3

### Characterization of cellulases

### CMC'ase assay

Assays for cellulase activity were performed using modified methods of the PAHBAH method (Lever M. Anal. Biochem. 1972, 47, 273-279 and Lever M. Anal. Biochem. 1977, 81, 21-27).

### Procedure

A test tube is filled with 250 µl 2.5% CMC in 50 mM glycine buffer pH 9 (CMC-low viscosity is purchased from Sigma) and 250 µl aliquots cellulase, diluted in the appropriate buffer. The test tube is incubated for 30 minutes at 40°C in a waterbath, whereafter 1.5 ml of a daily fresh prepared PAHBAH solution (1% PAHBAH in 100 ml 0.5 M NaOH with 100 µl bismuth solution (containing 48.5 g bismuth nitrate, 28.2 g potassium sodium tartrate and 12.0 g NaOH in 100 ml) is added. The mixture is heated at 70°C for 10 minutes, after which it is cooled on ice for 2 minutes. The absorption is measured at 410 nm. To eliminate the background absorbance of the enzyme samples a control experiment is executed as follows: a tube with substrate is incubated under the same conditions as the test tube. After the incubation 1.5 ml PAHBAH and the enzyme preparation is added (in this order). One unit (U) is defined as the amount of enzyme producing 1 µmol of glucose from CMC equivalent determined as reducing sugars per minute per gram product.

The buffer used for the determination of the pH/temperature profiles is a phosphate/citrate system. The pH/temperature profiles were determined using a fixed enzyme concentration which fits in the linear range of the dose response profile measured at pH 7 and 40°C. This enzyme concentration was used for the measurement of the activities under all other determined conditions.

The results for the cellulase produced by the BCE 103 clone are shown in Figure I. The cellulase produced by the BCE 103 clone shows good activities at alkaline pH, which makes it suitable for application in detergents with an alkaline pH.

### Example 4

### Anti redeposition test

### Procedure

20 ml 0.5% pigmented soil (fresh prepared, daily and consisting of 86% kaolin, 8% soot (from Degussa Flammruβ101), 4% iron oxide black and 2% iron oxide yellow (from Henkel Genthin GmbH)), in a detergent (Persil color without enzymes, 5 g/l, pH 8.5) was, under agitating (90 rpm) incubated with white cotton fabric (Windelbleiche, Krefeld, prewashed 5 cm diameter). Cellulase was added until a final concentration of 1 mU/ml. The mixture was incubated for 30 minutes at 40°C, 90 rpm. As a control the same incubation was carried out without the addition of cellulase. After the incubation the fabric was rinsed thoroughly with running cold water.

After drying the whiteness of the fabric was measured by remission (4 measurements per fabric) using a Micro color Dr. Lange Colorimeter. The control value was substracted from the sample value.

The results, expressed as delta Rem, are shown in Table 1.

### Fibre Damage Test

### Procedure:

One pad of cotton wool ('Wattenpads', 100% cotton, Warenhandels GmbH, Buchholz, Marke Olivia, Selling agency: Aldi) was incubated in 40 ml wash liquor (Persil color, without enzyme, 5 g/l pH 8.5), cellulase at a final concentration of 1 mU/ml was added in a sealed flask and incubated for 20 hours at 40°C under agitation (90 rpm). After the incubation, fibre damage was monitored by the measurement of the quantity of the reducing sugars in solution, using the PAHBAH method described in Example 3. As a control the same incubation was carried out without the addition of cellulase.

The results are shown in Table 1.

### Adsorption Test

### Procedure:

White cotton fabric (Windelbleiche, Bielefeld) prewashed with Persil without enzymes at 60°C, was cut round to 9 cm diameter (approx. 0.920 gram). One cotton swatch was incubated in 50 ml 50 mM glycine-NaOH buffer pH 9 including 0.1% SDS and 1 ml cellulase sample (600 mU/ml) for 60 minutes at 30°C. 2 ml samples were taken at T=0 and at T=60 minutes and were diluted directly (1:2) with 50 mM MES-buffer pH 6.5 and stored at 4°C until measurement. As control the same incubation was carried out without the addition of cotton textile. The activity measurement was determined with a PAHBAH method as described in Example 3, but at pH 6.5 in 50 mM MES buffer.

The adsorption was expressed as relative adsorption where the activity applied at the start of the experiment was set as 100%, T=0. $\text{100% activity value - remaining activity (%) = adsorption (%)}$.

The results are shown in Table 1.

**Table 1**

| results of the Antiredeposition Test, Fibre Damage Test and Adsorption Test | | | |
|---|---|---|---|
| Enzyme | Antiredeposition (delta REM) | Fibre Damage (mU) | Adsorption (%) |
| BCE 103 | 5.0 | 0.025 | 7 |
| Kao Kac® * | 7.5 | 0.006 | 0 |
| Denimax Ultra®MG ** | 1.2 | 0.155 | 36 |

| | | | |
|---|---|---|---|
| * Kao Kac® = cellulase of Kao Corporation, capable in cleaning performance but not in depilling performance. | | | |
| ** Denimax Ultra®MG = cellulase of Novo/Nordisk, capable in depilling performance but not in cleaning performance. | | | |

### Example 5

### Depilling test

Depilling and colour clarification performance were determined by treating pilled worn cotton fabric several times with cellulase. After washing the fabric was judged based on depilling and colour clarification compared to a control fabric which had not been treated with the enzyme.

One wash cycle consisted of the following steps: Four pilled and dark coloured cotton swatches (4x4 cm) were incubated in 40 ml wash liquor (5 g/l All color, pH 8.6) in a glass beaker (150 ml). The wash was performed at 40°C for 30 minutes in a shaked water bath (maximum shaking). After the incubation the fabrics were rinsed thoroughly for 10 minutes with running tap water and dried in a tumble dryer. Cellulase dosage is 2.5 mg/ml protein (BCA Pierce, BSA as standard).

As a control the same wash cycle was carried out without the addition of cellulase.

A total of 20 washing cycles were carried out. After every 5 wash cycles one fabric was taken out and a new one was added to the beaker in order to maintain the same fabric liquor ratio. After 20 washing cycles the fabrics were evaluated (on visual appearance) with the use of a panel, on a scale of 1 to 4, compared to the control, whereas:
1 = no depilling/colour clarification
2 = slight depilling/colour clarification
3 = good depilling/colour clarification
4 = very good depilling/colour clarification

The results are summarized in Table 3.

**Table 2**

| Results of the Depilling Test | |
|---|---|
| Sample | Colour clarification, depilling |
| BCE 103 | 4 |
| Kao Kac® * | 1 |
| Denimax Ultra®MG ** | 4 |

| | |
|---|---|
| *Kao Kac® = as described in Example 4 | |
| ** Denimax Ultra®MG = as described in Example 4 | |

### Example 6

### Desizing

A UniMac® Rotary Washer/Extractor-model UY 230 machine was loaded with 10 pounds of Swift Denim style #27261 (rigid, 100% cotton indigo dyed 14+ ounce per square yard denim). The Unimac was filled with hot water to a liquor ratio of 20:1 and heated to 82°C under slow rotation (5 rpm). 1.0 gram/liter Rapidstrip®102 (amylase) was added to the machine and the garments were allowed to soak for 3 minutes without rotation. Desizing took place for 15 minutes at 40 rpm.

After desizing the machine was drained and again filled with hot water to a liquor ratio of 20:1 and heated to 60°C under a low rotation (5 rpm). The garments were rinsed for 2 minutes after which the machine was drained. Again the machine was filled with hot water to a liquor ratio of 20:1 and heated to 55°C under low rotation (5 rpm). The garments were rinsed for 2 minutes after which the machine was drained.

### Stonewash

Directly after the desize procedure the machine was filled to a liquor ratio of 6:1 (30 liters) and was heated to a temperature desired for cellulase abrasion. 0.1 M K₂HPO₄ was added to the desired pH for abrasion. 72 mg Cellulase (BCE 103) was added to the machine and the garments were abraded for 60 minutes at 33 rpm, after which the machine was drained. After draining the machine was filled with hot water to a liquor ratio of 20:1 and heated to 60°C under a slow rotation (5 rpm). The garments were rinsed for 2 minutes after which the machine was drained. Again the machine was filled with hot water to a liquor ratio of 20:1 and heated to 37°C under a slow rotation (5 rpm). The garments were rinsed for 2 minutes after which the machine was drained and the garments tumbled dry.

The tested conditions were pH 7, 40°C and pH 7.5, 60°C. The results are shown in Table 3.

The jeans were evaluated (on visual appearance) on abrasion and indigo redeposition with the use of a panel, whereas for abrasion:
1] no abrasion
2] significant abrasion
for indigo redeposition:
1] acceptable level of indigo redeposition
2] unacceptable level of indigo redeposition

**Table 3**

| Results of stonewash test | | | | | |
|---|---|---|---|---|---|
| Sample | dosage | pH | Temp | Abrasion | indigo redeposition |
| BCE 103 | 72 mg | 7.0 | 40°C | 2 | 1 |
| BCE 103 | 72 mg | 7.5 | 60°C | 2 | 1 |
| No enzyme | - | 7.0 | 40°C | 1 | - |

## Claims

1. Cellulase which exhibit the following properties:
(a) show a delta REM of at least 4 units, preferably at least 5 units, in the Anti Redeposition Test, and
(b) show a depilling result which depilling result is at least comparable to that of the cellulase obtainable from CBS 670.93 in the Depilling Test.

2. A cellulase according to claim 1 which cellulase further exhibits
(c) a fibre damage of less than 0.05 milliunits in the Fibre Damage Test.

3. A cellulase according to any one of claims 1 or 2, which cellulase further exhibits
(d) an adsorption of less than 15% in the Adsorption Test.

4. A cellulase according to any one of claims 1 to 3, which is obtainable from a microorganism, preferably a fungus or a bacterium.

5. A cellulase according to claim 4 wherein the bacterium belongs to the genus Bacillus, preferably an alkalophilic Bacillus, more preferably Bacillus sp. CBS 670.93.

6. A cellulase according to any one of claims 1 to 5, wherein the cellulase has the amino acid sequence as listed in SEQ ID No. 2 or a derivative thereof.

7. An isolated DNA sequence encoding a cellulase according to any one of claims 1-6.

8. A vector capable of transforming a microbial host cell and characterized in that the vector comprises a DNA sequence according to claim 7.

9. A vector according to claim 8 and characterized in that the DNA sequence is operably linked to expression signals that ensure the expression of the DNA sequence in the microbial host.

10. A microbial host which contains a vector according to claims 8 or 9.

11. A microbial host according to claim 10 and characterized in that the microbial host expresses the DNA sequence.

12. A process for the preparation of the cellulase according to any one of claims 1 to 6, and characterized in that a microorganism producing the cellulase is cultivated in a suitable medium, whereafter the produced cellulase is recovered.

13. A detergent composition which comprises a cellulase according to any one of claims 1 to 6.

14. A detergent composition according to claim 13, wherein the detergent composition may be a granular or liquid detergent.

15. A detergent composition according to claim 13 or 14, wherein the detergent composition further comprises a surfactant and a builder.

16. The use of a detergent composition according to any one of claims 13 to 15.

17. The use of a cellulase according to any one of claims 1 to 6, in a textile treatment.

18. The use of a cellulase according to claim 17, wherein the textile treatment is a stone wash process or a biopolish process.

19. A fabric softener composition which comprises a cellulase according to any one of claims 1 to 6.

20. A fabric softener composition according to claim 19 wherein the fabric softener composition further comprises a cationic surfactant capable of providing fabric softening.

21. A depilling treatment composition which comprises a cellulase according to any one of claims 1 to 6.
